# EUROPÄISCHE PATENTSCHRIFT

(11) **EP 0 877 606 B1**
(45) Veröffentlichungstag und Bekanntmachung des Hinweises auf die Patenterteilung: **13.09.2000**
(21) Anmeldenummer: 97903327.1
(22) Anmeldetag: 19.02.1997
(51) Int. Cl.: A61K 9/20, A61K 31/19

(54) **IBUPROFEN-BRAUSEZUBEREITUNG SOWIE VERFAHREN ZUR HERSTELLUNG DERSELBEN**
EFFERVESCENT IBUPROFEN PREPARATION AND PROCESS FOR THE PRODUCTION THEREOF
PREPARATION EFFERVESCENTE D'IBUPROFENE ET SON PROCEDE DE PRODUCTION

(30) Priorität: 20.02.1996 DE 19606151
(43) Veröffentlichungstag der Anmeldung: 18.11.1998
(73) Patentinhaber: Losan Pharma GmbH, 79395 Neuenburg (DE)
(72) Erfinder: GRUBER, Peter, CH-4103 Bottmingen (CH)
(74) Vertreter: Becker Kurig Straus
(86) Internationale Anmeldenummer: EP9700789
(87) Internationale Veröffentlichungsnummer: WO9730698

(56) Entgegenhaltungen:
- EP-A- 0 181 564
- EP-A- 0 203 768
- EP-A- 0 351 353
- EP-A- 0 667 149
- WO-A-91/07174
- FR-A- 2 698 788

## Beschreibung

Die Erfindung betrifft den in den Patentansprüchen angegebenen Gegenstand.

Die Erfindung betrifft insbesondere eine neue, klar sich auf lösende Ibuprofen-Brausezubereitung sowie ein Verfahren zur Herstellung dieser Zubereitung.

Ibuprofen, oder (±)2-(4-Isobutylphenyl)-propionsäure, hat die folgende Strukturformel 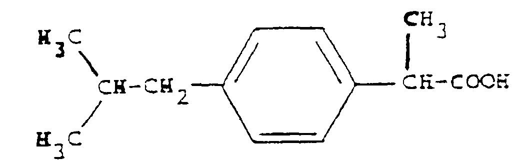 und ist ein seit Jahren bewährtes nicht steroidales Antiflogistikum aus der Gruppe der Phenylpropionsäurederivate, das sich über die Prostaglandinsynthesehemmung in den tierexperimentellen Entzündungsmodellen als wirksam erwiesen hat. In der Humantherapie reduziert Ibuprofen entzündlich bedingte Schmerzen, Schwellungen und Fieber. Es zeigt die für die nicht steroidalen Antirheumatika üblichen unerwünschten Nebenwirkungen.

Die pharmakokinetischen Eigenschaften der Substanz sind gut untersucht worden. In einigen klinischen Studien wurde auch gezeigt, daß Korrelation besteht zwischen dem Beginn und der Intensität des analgetischen Effektes und der Plasma-Ibuprofen--Konzentration. Im Falle von Ibuprofen hat Laska und Mitarbeiter gezeigt, daß besonders in den ersten zwei Stunden nach der Anwendung ein erhöhter Plasma-lbuprofen-Spiegel in einem erhöhten analgetischen Effekt resultiert. In einer anderen Studie zeigte sich, daß lösliches Ibuprofen einen früheren Beginn der Schmerzbefreiung zeigt als Ibuprofen-Tabletten. Auch bei Ibuprofen ist es offensichtlich, daß die galenische Formulierung einen großen Einfluß hat auf die Adsorptionsgeschwindigkeit und auf den Beginn des analgetischen Effektes. In einigen Studien wurde auch gezeigt, daß wirksame Ibuprofen-Plasma-Spiegel signifikant früher auftreten bei Anwendung von Ibuprofen-Lysinat im Vergleich zu Ibuprofen-Säure (G. Geisslinger et al. in Drug Invest. 5 (4), 238-242 (1994).

Wie aus der obigen Strukturformel hervorgeht, hat Ibuprofen ein asymmetrisches Kohlenstoffatom und liegt in der therapeutisch angewendeten Form als Razemat vor. Wie für zahlreiche Arzneimittelwirkstoffe mit einem oder mehreren asymmetrischen Kohlenstoffatomen bekannt ist, ist die eine enantiomere Form häufig sehr viel wirksamer. Es ist bekannt, daß R-(-)lbuprofen wesentlich weniger pharmakologisch aktiv ist als S-(+)Ibuprofen. Es wurde jedoch festgestellt, daß die S(+)-Form des Ibuprofens, also in Abwesenheit der R(-)-Form ein wesentlich grösseres pharmakologisches Potential besitzt, als zu vermuten war (siehe DE-OS 36 39 038).

Arzneimittel zur Schmerzbefreiung müssen jedoch preiswert sein. Die Situation im Augenblick ist so, daß wasserlösliche Salze wie Ibuprofen-Lysinat und Ibuprofen-Natrium wesentlich teurer sind als Ibuprofen-Säure selbst und andere weit verbreitete Analgetika wie Aspirin und Paracetamol.

Daher wurden große Anstrengungen unternommen, um Ibuprofen galenisch so zu formulieren, daß die billige Ibuprofen-Säure sich rasch und vollständig auflöst. Dies ist jedoch bislang nicht gelungen. Ibuprofen ist eine organische Säure mit einer schlechten Löslichkeit. Erst im pH-Bereich um 7 nimmt die Löslichkeit unter Salzbildung deutlich zu (pKs-Wert von Ibuprofen-Säure: 4,5). Aufgrund der enormen Verbreitung des Ibuprofens wäre es äusserst hilfreich, wenn durch die geschickte Wahl von Hilfsstoffen und ohne die Herstellung der Arzneimittel zu komplizieren, eine wesentlich schnellere und wenig pH-abhängige Auflösung der Ibuprofen-Säure möglich würde. Dies ist um so wichtiger, da Ibuprofen einen niedrigen Schmelzpunkt hat und nur schwer verarbeitet werden kann.

Der Geschmack der Ibuprofen-Säure ist sehr unangenehm und langanhaltend an den Schleimhäuten, die durch den Wirkstoff gereizt werden. Deshalb müssen orale feste Formen des Ibuprofens zu Filmtabletten oder in einigen Fällen sogar zu Dragees vearbeitet werden, um den Geschmack beim Schlucken zu kaschieren. Dies führt natürlich zu einer weiteren Verlängerung des Zerfalls und der Wirkstoffauflösung.

EP-A-0 228 164 (Boots) beschreibt eine Ibuprofen-Brausezubereitung, welche aber eine Suspension von Ibuprofen nach dem Abklingen der Brausereaktion ergibt. Die entstehende Suspension enthält den Wirkstoff in feinen Partikeln, der sich nach dem Trinken in der Schleimhaut der Mundhöhle teilweise festhaftet, und nach dem Schlucken der Lösung zu Brennen und lokalen Irritationen führt.

DE-A-36 38 414 beschreibt den Zusatz von Arginin oder Lysin in einer dem molaren Anteil übersteigenden Menge, um eine lösliche Form des Ibuprofens zu erhalten. Der Zusatz dieser Aminosäure-Komponenten bringt große Nachteile mit sich. Arginin und Lysin sind für die Verwendung als pharmazeutischer Hilfsstoff sehr teuer und übersteigen die Kosten für den Wirkstoff Ibuprofen bei weitem. Der Brausekörper enthält als Säurekomponente Natriumhydrogentartrat. Natriumhydrogentartrat wirkt als Säure so schwach, daß bei einem pH-Wert von über 6,5 für das gesamte System die Intensität der Brausereaktion gering ist. Somit wird nicht der von dem Patienten erwartete typische ansprechende Brauseeffekt erreicht.

EP-A-203 768 beschreibt eine Brausezusammensetzung, die Paracetamol, Acetylsalicylsäure oder Ibuprofen enthalten kann. Es wird hier vorgeschlagen, den Wirkstoff mit einem Granulationshilfsmittel (z.B. PVP) zu granulieren, dieses Granulat mit einem Teil einer Komponente des Brausegemisches zu vermischen und anschließend diese Vormischung mit einem Brausesystem zu vermengen. Die beschriebene Vorgehensweise eignet sich zur Herstellung klar löslicher Brausezubereitungen des Paracetamols und des Aspirins, nicht jedoch des Ibuprofens. Entsprechend dieser Vorgehensweise können keine 200 mg Ibuprofen enthaltende Brausezubereitungen hergestellt werden, die sich in 150 ml Wasser von ca. 20°C innerhalb von 2 Minuten auflösen und mehr als 90% gelöstes Ibuprofen enthalten. Um dies überhaupt erreichen zu können, müsste das Ibuprofen mit basischen Hilfsstoffen getrennt granuliert werden. Ansonsten liegt das Ibuprofen in ungelöster Form dispergiert in der Brausetablettenlösung vor, was aber nicht erwünscht ist.

EP-B-0 351 353 beschreibt Ibuprofen Brausetabletten, bei denen der Wirkstoff mit einer Zusammensetzung aus Natriumhydrogencarbonat und Zitronensäure hergestellt wird. Natriumhydrogencarbonat wird im Überschuß verwendet, um die Löslichkeit des Ibuprofens zu erhöhen. Es bestehen erhebliche Zweifel daran, ob durch diese einfache Zusammensetzung Brauselösungen entstehen, in denen der Wirkstoff sich vollkommen innerhalb der üblichen Zeit von wenigen Minuten auflöst. Alle Erfahrungen aus der Praxis zeigen, daß gelöstes Ibuprofen in Form seines Salzes gerade durch die relativ starke Zitronensäure wieder ausgefällt wird. Das ausgefällte Ibuprofen steigt als kleine Öltröpfchen an die Oberfläche des Wassers, sammelt sich dort und kristallisiert allmählich zu groben Kristallen, die nur sehr langsam wieder in Lösung gehen. Die in EP-A-0 351 353 beschriebene Herstellungsweise ist seit Jahrzehnten bekannt. So wird z.B. die Acetylsalicylsäure als Brausetablette hergestellt, indem man dem Brausekörper zusätzlich Natriumhydrogencarbonat zur besseren Auflösung der organischen ASS-Säure beifügt.

EP-A-369 228 (Bayer) beschreibt eine Ibuprofen-Brausezubereitung mit nachfolgender Zusammensetzung:
- 1 Gewichtsteil wasserlöslichem Ibuprofen-Salz,
- 2-10 Gewichtsteile Trägerstoff,
- 0,3-0,8 Gewichtsteile Stabilisator,
- 0,1-1 Gewichtsteile Natrium- oder Kaliumcarbonat.

Das Bayer-Verfahren zur Herstellung der obigen Zubereitung ist extrem teuer, weil das wasserlösliche Ibuprofen-Salz mit dem PVP und sehr viel Wasser erst einmal in eine klare Lösung überführt werden muß. Diese klare Lösung wird in einem Wirbelschichtgranulator auf Natriumhydrogencarbonat aufgesprüht. Anschließend muß dieses Granulat mit Natriumcarbonat, das wiederum in einer großen Menge Wasser aufgelöst wird, überzogen werden. Soll dieses Granulat zu Brausetabletten verarbeitet werden, muß es gemäß EP-A-0 369 228 nochmals mit einer wäßrigen Dinatriumfumaratlösung besprüht werden. Diese Vorgänge sind sehr teuer, erfordern stundeslanges Sprühen bei hohen Temperaturen. Natriumcarbonat, gelöst in Wasser, neigt dazu, das Wasser infolge Hydratbildung extrem schwer abzugeben. Dadurch ist der Trocknungsvorgang besonders lang und erfordert hohe Temperaturen. Bei dieser Herstellungsweise ist nicht ausgeschlossen, daß gelb verfärbte Granulate entstehen. Berechnet man die Menge an Wasser, die zur Herstellung von Ibuprofengranulat für 100.000 Tabletten (siehe Beispiel 1 und 2 der EP-A-369 228) benötigt wird, so errechnet sich die immense Menge von 230 kg Wasser, die bei 100°C Zulufttemperatur zu verdampfen sind.

Wie später entsprechend der erfindungsgemäßen Herstellung noch gezeigt werden wird, benötigt man für die Herstellung der gleichen Menge des erfindungsgemäßen Granulats 3,5 kg Wasser und 3 bis 5 kg Ethanol. Damit ist der Trocknungsprozeß entscheidend wirtschaftlich günstiger und schonender.

EP-A-0 667 149 beschreibt eine Ibuprofen-Brausetablette, die neben Natriumhydrogencarbonat (3,5 bis 12 Teile) auch Kaliumhydrogencarbonat (3 bis 9 Teile), jeweils bezogen auf 2 Teile Ibuprofen, enthalten muß. Gemäß EP-A-0 667 149 wird kein Brausekörper hergestellt, sondern die gesamte Menge an Bicarbonat mit dem Wirkstoff granuliert und ein eigenes Säuregranulat mit einem Süßstoff und eine Zuckeraustauschstoff hergestellt. Es bestehen auch hier erhebliche Zweifel daran, ob durch diese einfache Zusammensetzung Brauselösungen entstehen, in denen sich der Wirkstoff vollkommen innerhalb der üblichen Zeit von wenigen Minuten auflöst. Wie die Erfahrung zeigt, wird gelöstes Ibuprofen in Form seines Salzes gerade durch die Säure wieder ausgefällt und steigt dann in Form von kleinen Öltröpfchen an die Oberfläche des Wassers, sammelt sich dort und kristallisiert allmählich zu groben Kristallen, die entweder überhaupt nicht oder nur sehr langsam wieder in Lösung gehen.

CH-A-684 929 beschreibt Ibuprofen-haltige Brausezusammensetzungen. Der Wirkstoff wird hier in Form des Natrium- oder Kaliumsalzes eingesetzt. Die Zusammensetzung muß Siliciumdioxid enthalten, welches unlöslich ist und zu einer trüben Lösung führt. Dies ist jedoch bei Analgetika unerwünscht; man erwartet die Auflösung der Tablette zu einer klaren Lösung, wie es beispielsweise bei Aspirin-Brausetabletten schon lange Stand der Technik ist. Weiterhin muß die in der CH-A-684 929 beschriebene Brausezusammensetzung einen Celluloseether und ein Tensid enthalten, damit das ausgefallene Ibuprofen wieder besser in Lösung geht. Insgesamt ist aus der CH-A-684 929 zu entnehmen, daß die Ausfällung des Ibuprofens nicht vermieden werden kann und viele Maßnahmen getroffen werden müssen, um das Wiederauflösen des Ibuprofens zu erreichen. Die beschriebene Ibuprofen-haltige Brausezusammensetzung enthält ausserdem fast 1000 mg Kalium- und Natriumcarbonat, was zu schlechten organoleptischen Eigenschaften führt.

EP-A-0 181 564 beschreibt eine Ibuprofen-Brausezubereitung, wobei die Ibuprofen-Partikel mit einem Schleimstoff wie Xanthan und/oder Maltodextrin und Fumarsäure umhüllt und zu einem Granulat verarbeitet werden. Nach Aufmahlung des Granulats wird dieses zusammen mit einer weiteren Säure wie z.B. Zitronensäure auf Zucker aufgezogen. Dieses Granulat kann auch mit einer Brausemischung aus Zitronensäure und Calciumcarbonat gemischt werden. Beim Auflösen der Brausemischung gemäß EP-A-0 181 564 in Wasser wird der Wirkstoff Ibuprofen nicht gelöst und bildet in Wasser eine Suspension. Die Ibuprofen-Partikel können daher an Mund- und Rachenschleimhaut hängen bleiben und verursachen die allgemein bekannten Schleimhautreizungen beim Trinken dieser Suspension.

Die Analgetikapreise sind weltweit sehr niedrig. Die Darstellung einer Wirkstoffzubereitung als Brausetablette ist noch teurer, weil das Gewicht der Tablette in der Größenordnung von 3 g liegen muß und hohe Kosten an Packmitteln und hohe Produktionskosten entstehen. Die Realisierung einer wirtschaftlich sehr günstig herstellbaren Ibuprofen Brausetablette ist somit sehr vordringlich.

Daher ist es Aufgabe der Erfindung, eine neuartige Ibuprofen-Brausezubereitung, die klar gelösten Wirkstoff ergeben soll, sowie ein Verfahren zu ihrer Herstellung, bereitzustellen.

Diese Aufgabe wird gelöst durch die erfindungsgemäße Ibuprofen-Brausezubereitung, die zwei getrennt hergestellte Granulate enthält:
(a) ein Ibuprofengranulat, hergestellt aus Ibuprofensäure und einem basischen Hilfsstoff in einem Molverhältnis von einem Mol Ibuprofensäure auf 0,5 bis 10 Mol basischen Hilfsstoffs und
(b) ein Ibuprofen-freies Brausegranulat, das eine Säurekomponente und eine Kohlendioxid-bildende Komponente enthält.
70% des basischen Ibuprofengranulats (a) liegen in einer Korngröße im Bereich zwischen 0,1 und 2,0 mm, insbesondere in einem Bereich von 0,1 und 1,25 mm, vor. Die erfindungsgemäße Ibuprofen-Brausezubereitung weist ein Mol-Verhältnis von Ibuprofensäure zu der Säurekomponente des Brausegranulats (b) im Bereich von einem Mol Ibuprofensäure auf 0,5 bis 6 Mol Säurekomponente auf. Das Mol Verhältnis der Ibuprofensäure zu der Kohlendioxid-bildenden Komponente des Brausegranulats (b) liegt im Bereich von einem Mol Ibuprofensäure auf 1 bis 30 Mol der Kohlendioxid-bildenden Komponente. Erfindungswesentlich ist, daß sich das Brausegranulat (b) bei Zerfall der Ibuprofen-Brausezubereitung in Wasser sofort auflöst, um (etwas grössere) Ibuprofengranulatteilchen (a) freizugeben, aus welchen dann nachfolgend das Ibuprofen in Lösung geht.

Die Erfindung betrifft weiterhin ein Verfahren zur Herstellung einer Ibuprofen-Brausezubereitung, gemäß welchem man aus Ibuprofensäure und mindestens einem basischen Hilfsstoff und gegebenenfalls weiteren wasserlöslichen Hilfsstoffen ein Ibuprofengranulat (a) herstellt, getrennt davon ein Ibuprofen-freies Brausekörpergranulat (b) herstellt, die beiden Komponenten (a) und (b) mit üblichen weiteren Hilfsstoffen vermischt und zu Brausetabletten verpreßt oder die Brausezubereitung in Sachets abfüllt.

In den Unteransprüchen sind vorteilhafte Ausführungsformen der Erfindung enthalten.

Es wurde nun völlig überraschend gefunden, daß mit äusserst einfachen Mitteln die Auflösegeschwindigkeit des Wirkstoffes Ibuprofensäure und die starke pH-Abhängigkeit der Löslichkeit verbessert werden kann, wenn man den Wirkstoff dicht mit basischen Hilfsstoffen verbindet. Hierfür sind insbesondere anorganische basische Hilfsstoffe wie Natriumcarbonat, Kaliumcarbonat, Natriumbicarbonat, Kaliumbicarbonat, basische Phosphate wie Trinatriumphosphat bestens geeignet. Alle diese Hilfsstoffe zeichnen sich durch einen äusserst günstigen Preis aus und erschweren in keinem Fall die Herstellung von Granulaten, die letztlich sogar zu Brausetabletten weiterverarbeitet werden.

In einer bemerkenswert einfachen Herstellungsweise wird entsprechend der Erfindung in einem Feuchtmischgerät Ibuprofensäure mit billigen basischen Hilfsstoffen wie Natriumcarbonat, Kaliumcarbonat, Kaliumhydrogencarbonat, Natriumhydrogencarbonat, Trinatriumcitrat, Trinatriumphosphat in einem molaren Verhältnis von einem Mol Ibuprofen-Säure auf 0,5 bis 10 Mol, bevorzugt ein Mol Ibuprofen-Säure auf 1,0 bis 3 Mol basischer Komponente befeuchtet.

Ibuprofen im Rahmen der vorliegenden Erfindung liegt entweder als Racemat, als racemisches Gemisch mit seinen Enantiomeren, als Pseudoracemat, d.h. als Mischung von gleichen Anteilen S- und R-lbuprofen oder in Mischung unterschiedliche Anteile von S- und R-lbuprofen im Bereich zwischen reinem S- und reinem R-lbuprofen vor. Der Fachmann weiß, daß für die S-Form von Ibuprofen jeweils nur die Hälfte der angegebenen Mengen für das Racemat, d.h. 100 bis 200 mg bei Brausetabletten und 100 bis 400 mg bei Brausegranulaten eingesetzt werden müssen.

Die erfindungsgemäße Brausezubereitung liegt insbesondere in Form einer Brausetablette vor, die 200 bis 800 mg racemisches Ibuprofen oder 100 bis 200 mg S-Ibuprofen enthält. In einer bevorzugten Ausführungsform enthält die erfindungsgemäße Brausetablette 200 bis 400 mg racemisches Ibuprofen. In einer anderen Ausführungsform der Erfindung kann die Brausezubereitung in Form von in Sachets abgefülltem Granulat vorliegen, wobei sie 200 bis 800 mg racemisches Ibuprofen oder 100 bis 400 mg S-Ibuprofen enthält. Bevorzugt ist, daß die Sachets 200 bis 800 mg racemisches Ibuprofen enthalten.

Nach Auflösen von 100 bis 400 mg S-Ibuprofen oder 200 bis 800 mg racemischem Ibuprofen beträgt der pH-Wert zwischen 6,5 und 9,0, was einer entsprechenden Menge der Brausezubereitung in 150 ml Wasser entspricht.

Als geeignete basische Hilfsstoffe kommen erfindungsgemäß insbesondere folgende Substanzen aus der Gruppe aus NaHCO₃, KHCO₃, Na₂CO₃, K₂CO₃, Na₃PO₄, Natriumcitrat, K₂O, Na₂O, CaO, MgO,
Ca(OH)₂, Mg(OH)₂, basisches Magnesiumcarbonat in Betracht, wobei Na₂CO₃, K₂CO₃, Na₃PO₄, NaHCO₃ KHCO₃ oder Gemische davon besonders bevorzugt sind.

Als Befeuchtungsmittel können Wasser oder Wasser-Alkohol-Mischungen wie Wasser mit Ethanol und Isopropanol eingesetzt werden. Besonders gut eignen sich Mischungen aus Alkohol mit Wasser im Gewichtsverhältnis 2:8 bis 8:2. Dabei entstehen auch ohne den Zusatz eines Klebstoffes schön strukturierte Granulate, die nach der Trocknung gesiebt werden. Besonders vorteilhaft kann diese einfache Granulation in einem Vakuumgranulator erfolgen, in dem das Mischen, das Befeuchten und das Trocknen im selben Gerät durchgeführt werden. Ansätze in der Größenordnung von 500 kg lassen sich innerhalb von 2 bis 4 Stunden bei einer Wandtemperatur von 60°C ausreichend trocknen. Die Granulate sind durch den Zusatz der basischen Hilfsstoffe völlig wasserlöslich. Die Erzeugung einer klaren Lösung durch ein derart einfach herstellbares Granulat war völlig überraschend. Die Herstellung solcher Granulate ist wesentlich billiger als der Einsatz von Ibuprofen-Aminosäuregranulaten des Arginins oder Lysins bzw. von Ibuprofen-Natriumsalzen.

Die erfindungsgemäße Brausezubereitung enthält neben dem Wirkstoffgranulat einen getrennt hergestellten Brausekörper. Bekanntlich wird das gelöste Ibuprofen leicht wieder durch die Säure des Brausekörpers zur Ibuprofen-Säure neutralisiert. Dieses Ibuprofen ist unlöslich, steigt als winzige Öltröpfchen an die Wasseroberfläche beim Auflösen der Brausezubereitung und erstarrt dort zu schleimhautreizenden groben Ibuprofen-Kristallen. Dies wird durch zwei weitere erfindungswesentliche Schritte vermieden:
- Die in jedem Brausekörper notwendige Säure wird direkt und innig mit dem neutralisierenden CO₂-bildenden KHCO₃, NaHCO₃ und Natriumglycincarbonat verbunden. Der entstehende Brausekörper löst sich bei Kontakt mit Wasser nahezu spontan in wenigen Sekunden auf, wobei die direkt mit der Säure verbundenen Stoffe NaHCO₃, KHCO₃ und Natriumglycincarbonat schlagartig die Säure abpuffern. Die Lösungsgeschwindigkeit des Brausekörpers in Wasser von 20°C beträgt maximal 60 Sekunden.
- Ibuprofen wird in ein gut strukturiertes, mechanisch stabiles Granulat überführt. Mindestens 70% der Teilchen haben eine Größe zwischen 0,1-1,25 mm, bevorzugt 0,1-0,5 mm. Die Lösungsgeschwindigkeit der Wirkstoffgranulate beträgt 50 Sekunden bis 200 Sekunden in entmineralisiertem Wasser von 20°C.

Die zeitlich versetzte zweistufige Auflösung beider Granulate ist erfindungswesentlich. Durch die rasche Auflösung der Brausekörper ist bereits die Hauptmenge der Säure neutralisiert, bevor das Ibuprofen in Lösung geht. Der zweistufige Auflösungsprozeß der Brausezubereitung ist bei der Anwendung mit Wasser deutlich zu erkennen: Bei der Auflösung der Brausetablette fallen Wirkstoffgranulatkörner aus der schwimmenden Tablette heraus, fallen zu Boden und gehen deutlich zeitversetzt in Lösung.

Die zeitlich versetzte Auflösung des Wirkstoffgranulates kann in vielfältiger Weise wie folgt gesteuert werden:
- Verhältnis Wasser/Ethanol bei der Granulation zu deren Menge
- Intensität des Knetens während der Befeuchtung
- Siebgröße beim Feuchtsieben und Trockensieben des Granulates.

Schließlich können bei der Granulation neben dem Wirkstoff und dem basischen Hilfsstoff lösungssteuernde, pharmazeutisch weit verbreitete wasserlösliche Hilfsstoffe wie Lactose, Mannit, Sorbit, Xylit, Glycin, Gelatine eingesetzt werden. Die basischen Hilfsstoffe können in dem angegebenen molaren Verhältnis auch aus Mischungen bestehen, wie z.B. als NaHCO₃ oder Na₂CO₃ oder aus K₂CO₃ und Trinatriumcitrat.

Der Brausekörper wird mit den üblichen Komponenten in bekannter Weise hergestellt. Die Säurekomponente besteht beispielsweise aus Zitronensäure, Weinsäure, Apfelsäure, Adipinsäure, Ascorbinsäure und Mononatriumcitrat. Bevorzugt wird letzteres eingesetzt. Besonders bevorzugt wird Natriumdihydrogencitrat eingesetzt. Auch Mischungen der genannten Komponenten sind denkbar. Die CO₂-bildende Komponente besteht aus NaHCO₃, KHCO₃, Na₂CO₃, K₂CO₃ und Natriumglycincarbonat, bevorzugt NaHCO₃. Der Brausekörper kann noch weitere Komponenten enthalten wie Xylil, Sorbit, Natriumsulfat und Bindemittel wie Gelatine, Polyvinylpyrrolidon und Methylcellulose.

Die Brausezubereitung enthält weitere Hilfsstoffe wie Sußstoffe und/oder Zuckeraustauschstoffe und/oder Aromen.

Die Brausezubereitung enthält daher Hilfsstoffe wie Saccharin-Natrium, Natriumcyclamat, Aspartam und Trockenaromen. Die erfindungsgemäße Zubereitung wird als Brausegranulat in Beutel abgefüllt oder zu Tabletten mit einem Gewicht von ca. 3-4 g abgepreßt und in Röhren abgefüllt und mit einem Trockenstopfen verschlossen.

Die erfindungswesentliche zweistufige Auflösung beider Granulate kann am besten in einer in vitro-Freigabeapparatur mit einem Blattrührer geprüft werden. Die genannte Apparatur ist in der europäischen oder deutschen Pharmakopoe (DAB10) genau beschrieben.
Bedingungen:
500 ml Wasser, entmineralisiert
Temperatur: 20°C
Umdrehungsgeschwindigkeit: 100 UpM
Probemenge: 1 g Wirkstoffgranulat oder Brausekörpergranulat
Auflösung Brausekörpergranulat: max. 60 Sekunden
Auflösung Wirkstoffgranulat: 50-200 Sekunden.

Das erfindungsgemäße Granulat geht von Ibuprofen-Säure aus und nicht von wasserlöslichem Ibuprofen-Salz. Das erfindungsgemäße Granulat enthält weiterhin keinen Stabilisator.

Gemäß EP-A-0 369 228 (Bayer) wird ausdrücklich beschrieben, daß Ibuprofen in Form seiner wasserlöslichen Salze mit dem Trägerstoff und dem Stabilisator granuliert wird. Diesem Granulat wird später eine Säurekomponente zugemischt.

Bei dem erfindungsgemäßen Granulat wird ein Brausekörper zugemischt. Das bei dem erfindungsgemäßen Granulat eingesetzte PVP im Brausekörper hat eindeutig die Funktion eines Klebstoffes für den Brausekörper und ist nicht zur Stabilisierung des gelösten Ibuprofens gedacht, wie es ausdrücklich in dem Bayer-Patent beschrieben wird.

Bei einer besonders vorteilhaften Ausführungsform der Erfindung wird ein Mol Ibuprofen mit 2,6 Mol Natriumcarbonat mit einem Gemisch aus Wasser/Ethanol 5:5 granuliert. Nach dem Trocknen und Sieben entsteht ein gut strukturiertes Granulat mit einer Korngrößenverteilung im wesentlichen zwischen 0,1 und 0,5 mm. Weniger als 30% des Granulates sind < 0,1 mm. Die für jede Brausetablette notwendige Säure wird mit dem kohlendioxydbildenden Agens (Natriumhydrogencarbonat oder Kaliumhydrogencarbonat) in einem getrennten Granulationsschritt hergestellt. Als Säurekomponente hat sich besonders Mononatriumcitrat bewährt, da es weniger sauer ist als Zitronensäure. Bei der Herstellung des Brausekörpers werden üblicherweise Bindemittel, wie z.B. Polyvinylpyrolidon, eingesetzt. Die Granulierung erfolgt vorteilhaft mit Ethanol oder Isopropanol mit geringem Zusatz von Wasser. Dieses kann strukturfördernde Hilfsstoffe wie Sorbit und Xylit enthalten. Neben dem Wirkstoffgranulat und dem Brausekörper-Granulat werden noch Süßstoffe und Aromastoffe zugesetzt. Wie in den Beispielen ausgeführt, entsteht eine Brausetablette, die eine Zerfallszeit zwischen einer und 2,5 Minuten hat, 200 mg Ibuprofen enthält und den Wirkstoff nach dem Auflösen in völlig gelöster Form enthält. Bei der Auflösung der Brausetablette fällt ein zweiter erfindungswesentlicher Schritt auf:

Die Brausetablette arbeitet in der Art einer 2-Stufen-Brausetablette, in dem bei Zutritt des Wassers der Brausekörper sehr schnell reagiert und es durch die Reaktion zwischen der Säure und dem Natriumhydrogencarbonat zu einer starken Kohlendioxidbildung kommt und die Säure durch das im Überschuß vorhandene Natriumhydrogencarbonat sehr rasch abgepuffert wird. Man sieht wie aus der verbleibenden Brausetablette Granulatkörner, bestehend aus Ibuprofen und Natriumcarbonat herausfallen und sich erst in einer zweiten Stufe auflösen. Dadurch erreicht man folgenden erfindungswesentlichen Schritt:

Die Säure wird durch das sofort abpuffernde Natriumhydrogencarbonat neutralisiert und kann bereits in Lösung gegangenes Ibuprofen nicht wieder ausfällen. Die Freisetzung des Ibuprofens als gelöstes Ibuprofen-Salz ist verzögert, weil sich die Partikel des Wirkstoffgranulates langsamer auflösen als die Partikel des Brausekörpers. Somit geht das Ibuprofen im wesentlichen erst dann in Lösung, wenn die Säure bereits durch das Bicarbonat abgepuffert ist. Durch diesen erfindungswesentlichen Schritt erreicht man, daß die Säure praktisch kein gelöstes Ibuprofen während des Auflösens der Brausetablette wieder ausfällen kann.

Das Ibuprofen-Granulat besteht im wesentlichen aus dem Wirkstoff und den genannten basischen Hilfsstoffen. Zur Steuerung der Wirkstofffreisetzung, d.h. zur Auflösung der Granulate, kann dem Wirkstoffgranulat aber auch gewisse Anteile an leicht wasserlöslichen Hilfsstoffen, wie Glycin, Sorbit, Mannit, Saccharose zugesetzt werden.

Die Erfindung wird nun anhand der folgenden Beispiele näher erläutert werden:

### Beispiel 1:

| a) | Wirkstoffgranulat | mg/Dosis | kg/100.000 Anwendungen |
|---|---|---|---|
| | Ibuprofen | 200 mg | 20,0 kg |
| | Kaliumcarbonat | 200 mg | 20,0 kg |
| | Natriumhydrogencarbonat | 600 mg | 60,0 kg |

In einem Vakuumgranulierer wurden 20,0 kg Ibuprofen, 20,0 kg K₂CO₃ und 60,0 kg NaHCO₃ kräftig gemischt und mit einer Lösung aus 4 kg Wasser und 6 kg Ethanol unter Rühren besprüht. Sobald sich eine deutliche Struktur gebildet hat, wird die Kesselwand auf 60°C beheizt und das Granulat innerhalb 2 Stunden getrocknet. Das getrocknete Granulat siebt man durch ein Sieb mit der Maschenweite 1,0 mm. Das Granulat reicht-für 100.000 Anwendungen.

| b) | Brausekörper | mg/Dosis | kg/100.000 Anwendungen |
|---|---|---|---|
| | Natriumdihydrogencitrat | 700 mg | 70,0 kg |
| | Natriumhydrogencarbonat | 1040 mg | 104,0 kg |
| | Mannit | 200 mg | 20,0 kg |
| | Polyvinylpyrolidon | 20 mg | 2,0 kg |

Die genannten Mengen werden in einem Vakuumgranulierer gemischt und mit 16 kg 90%igem Ethanol befeuchtet. Das Granulat wird bei 60°C Wandtemperatur des Kessels in 1,5 Stunden getrocknet und durch Maschenweite 1,25 mm gesiebt.

Die Auflösegeschwindigkeiten betragen

| | |
|---|---|
| Wirkstoffgranulat | 108 Sekunden |
| Brausekörpergranulat | 48 Sekunden. |

Beide Granulate werden gemischt, Aspartam und Aroma zugefügt und in Beutel abgefüllt. Jeder Beutel enthält die 200 mg Ibuprofen entsprechende Menge Wirkstoffgranulat.

Beim Auflösen in 150 ml Wasser entsteht nach 2 Minuten eine klare Lösung. Der pH-Wert der Lösung beträgt 6,9.

### Beispiel 2

| a) | Wirkstoffgranulat | mg/Dosis | kg/100.000 Anwendungen |
|---|---|---|---|
| | Ibuprofen | 200 mg | 20,0 kg |
| | Na₂CO₃ | 275 mg | 27,5 kg |
| | Glycin | 200 mg | 20,0 kg |

Wie in Beispiel 1 beschrieben, werden die drei Komponenten in einem Vakuumgranulierer gut gemischt und mit einer Lösung aus 3,5 kg Wasser und 3,5 kg Ethanol besprüht. Bei kräftigem Rühren entsteht eine ausgeprägte Granulatsstruktur.

Trocknung siehe Beispiel 1
Trockensiebung 1,00 mm, dann 0,6 mm

| | |
|---|---|
| Korngrößenverteilung | 12% < 0,1 mm |
| | 88% < 0,6 mm. |

| b) | Brausekörper | mg/Dosis | kg/100.000 Anwendungen |
|---|---|---|---|
| | Natriumhydrogencarbonat | 1640 mg | 164,0 kg |
| | Natriumdihydrogencitrat | 720 mg | 72,0 kg |
| | Sorbit | 100 mg | 10,0 kg |
| | Aspartam | 30 mg | 3,0 kg |
| | Saccharin-Natrium | 10 mg | 1,0 kg |
| | Polyvinylpyrolidon | 20 mg | 2,0 kg. |

Wie in Beispiel 1 beschrieben, werden die gemischten Komponenten mit 25 kg einer 8%igen isopropanolischen Polyvinylpyrolidonlösung besprüht, danach mit 7,1 kg einer 70%igen Sorbitlösung. Nach der Trocknung wird das Granulat durch 1,25 mm gesiebt.

Die Auflösegeschwindigkeiten betragen:

| | |
|---|---|
| Brausekörpergranulat | 50 Sekunden |
| Wirkstoffgranulat | 130 Sekunden |

Beide Granulate werden mit 5,5 kg Zitronenaroma gemischt und zu Brausetabletten mit 3,3 g Gewicht verpreßt.

Die Tablette zerfällt in 95 Sekunden in 150 ml Wasser von 20°C und nach 150 Sekunden sind alle Komponenten klar gelöst. Der pH-Wert beträgt 7,0.

### Beispiel 3:

| a) | Wirkstoffgranulat | mg/Dosis | kg/100.000 Anwendungen |
|---|---|---|---|
| | Ibuprofen | 800 mg | 80,0 kg |
| | Na₂CO₃ | 210 mg | 21,0 kg |

Wie in Beispiel 1 beschrieben, werden die zwei Komponenten in einem Vakuumgranulierer gut gemischt und mit einer Lösung aus 7 kg Wasser und 3 kg Isopropanol besprüht. Beikräftigem Rühren entsteht eine ausgeprägte Granulatstruktur. Trocknung siehe Beispiel 1.

| | |
|---|---|
| Trockensiebung | 1,00 mm |
| Korngrößenverteilung | 27% < 0,1 mm |
| | 73% < 1,0 mm |

| b) | Brausekörper | mg/Dosis | kg/100.000 Anwendungen |
|---|---|---|---|
| | Natriumhydrogencarbonat | 1000 mg | |
| | Zitronensäure | 400 mg | 100,0 kg |
| | Saccharose | 2000 mg | 200,0 kg |
| | Saccharin-Natrium | 15 mg | 1,5 kg |
| | Sorbit | 85 mg | 8,5 kg |

Wie in Beispiel 1 beschrieben, werden die gemischten Komponenten mit 25 kg Isopropanol und anschließend mit 11,3 kg einer wäßrigen 75%igen Sorbitlösung befeuchtet.
Nach der Trocknung wird das Granulat durch 0,71 mm gesiebt.

Die Auflösegeschwindigkeiten betragen:

| | |
|---|---|
| Wirkstoffgranulat | 154 Sekunden |
| Brausekörpergranulat | 58 Sekunden |

Beide Granulate werden gemischt, Pfefferminzaroma zugefügt und in Beutel abgefüllt. Jeder Beutel enthält die 800 mg Ibuprofen entsprechende Menge Granulat. Beim Auflösen in 200 ml Wasser entsteht nach 3 Minuten eine klare Lösung. Unter Beibehaltung der angegebenen Menge Brausekörper können jedoch auch Beutel abgefüllt werden, die 200, 400, 600 mg Ibuprofen enthalten.

### Vergleichsbeispiel 1 (gemäß EP-A-369 228; Beispiel 2)

Es wurde exakt Beispiel 2 der EP-A-369 228 nachgearbeitet.

Ein Ibuprofen-Brausegranulat, mit welchem 200 mg Ibuprofen zu applizieren sind, ist wie folgt zusammengesetzt:

| | | |
|---|---|---|
| 1) | Ibuprofen-Natriumsalz | 0,443 kg |
| 2) | Polyvinylpyrrolidon | 0,160 kg |
| 3) | Natriumhydrogencarbonat | 2,700 kg |
| 4) | Wasser | 1,565 kg |

### Ansatzgröße: 2000 Brausetabletten.

Aus den Komponenten 1), 2) und 4) wird eine Lösung hergestellt, die auf das in einer Wirbelschichtapparatur befindliche Natriumhydrogencarbonat aufgesprüht wird.

| | |
|---|---|
| Zulufttemperatur | 90°C |
| Sprühdruck | 1,2 bar |
| Sprühzeit | 30 Minuten |

Es entsteht ein gut strukturiertes Granulat. Dieses wird mit einer Lösung aus

| | |
|---|---|
| Natriumcarbonat | 0,300 kg und |
| Wasser | 1,675 kg |

unter gleichen Bedingungen wie oben besprüht.
Die Sprühzeit beträgt 32 Minuten.
Nach kurzer Trocknung wird das Granulat mit einer Lösung aus

| | |
|---|---|
| Dinatriumfumarat | 0,300 kg und |
| Wasser | 2,000 kg |

unter den Bedingungen wie oben besprüht.
Die Sprühzeit war 38 Minuten.
Die Trocknung betrug 4 Stunden bei 55°C im Umlufttrockenschrank. Das Granulat ist weiß, gut strukturiert und gut fließfähig. Der Trocknungsverlust bei 70°C liegt unter 1%.
Die Tablettierung erfolgt nun mit
1,951 kg Granulat und
0,549 kg Zitronensäure.
Die beiden Komponenten werden 20 Minuten miteinander gemischt. Diese Mischung wird zu Tabletten verpreßt mit einem Durchmesser von 25 mm und einer Bruchfestigkeit von 60-90 N.

### Versuchsergebnis

In 100 ml Wasser von 20°C zerfallen die Tabletten gemäß EP-A-0369 228 innerhalb von 2 bis 3,5 Minuten unter starker Ausfällung von Ibuprofen. In einer Art von Schaum sammelt sich das ölig ausgefallene, halbkristalline Ibuprofen an der Oberfläche und setzt sich als ein halbkristalliner weißer Rand an der Glaswand ab. Trinkt man diese trübe Lösung mit den ausgefällten Ibuprofen, so entsteht nach ca. 30 Sekunden das bekannte, absolut untolerable Brennen von ungelöster Ibuprofensäure an den Mundschleimhäuten. Rührt man die trübe Lösung ca. 6-8 Minuten, löst sich das ausgefällte Ibuprofen. Es entsteht eine klare Lösung mit einem pH-Wert von 6,8.

### Bewertung:

Die Brausetablette zerfällt zwar wie angegeben in ca. 2-3, 5 Minuten. Es entsteht jedoch eine trübe Lösung, auf deren Oberfläche große Mengen ausgefällten Ibuprofens schwimmen. Diese Suspension ist nicht trinkbar. Eine Auflösung des ausgefällten Ibuprofens unter Rühren nach 6-8 Minuten ist nicht tolerabel. Brausetabletten müssen sich praktisch von selbst innerhalb von ca. 3 Minuten klar oder fast klar auflösen.

Obwohl sich das Granulat vor der Zugabe der Zitronensäure vollkommen klar auflöst, führt die Zugabe von Zitronensäure zu einer spontanen, offensichtlich unvermeidbaren Ausfällung von bereits gelöstem Ibuprofen.

### Vergleichsbeispiel 2 (gemäß EP-A-0 667 149; Beispiel 1)

Es wurde exakt Beispiel 1 von EP-A-667 149 nachgearbeitet.

Es wird entsprechend der Herstellungsweise wie in Beispiel 1 der EP-A-667 149 beschrieben ist, ein basisches Granulat mit folgender Zusammensetzung hergestellt (Ansatzgröße 2000 Tabletten).

| | | |
|---|---|---|
| 1) | Ibuprofen | 0,400 kg |
| 2) | Natriumhydrogencarbonat | 1,000 kg |
| 3) | Kaliumhydrogencarbonat | 1,400 kg |
| 4) | Sucrosemonopalmitat | 0,005 kg |
| 5) | Wasser | 1,000 kg |

Exakt nach Beispiel 1 von EP-A-667 149 wird aus den Komponenten 4) und 5) eine Lösung hergestellt, die auf die in einer Wirbelschichtapparatur befindliche gesiebte Mischung aus den Komponenten 1), 2) und 3) aufgesprüht wird.

| | |
|---|---|
| Zulufttemperatur | 60°C |
| Sprühdruck | 1,0 bar |
| Sprühzeit | 15 Minuten |
| Trocknung | 20 Minuten |
| Trocknungsverlust | 0,6 % bei 70°C. |

Das Granulat ist gut strukturiert und fließfähig und löst sich in demineralisiertem Wasser klar auf.

| | |
|---|---|
| Unter den beschriebenen Bedingungen wird die | |
| Zitronensäure | 1,080 kg |
| separat mit der wässrigen Lösung von | |
| Aspartam und | 0,140 kg |
| Sorbit | 0,400 kg |
| granuliert und getrocknet. | |
| Trocknungsverlust: | 0,3 % bei 70°C. |

Die zwei Granulate, gesiebt durch 1,0 mm, werden analog Beispiel 1 mit 67 g Orangen-Trockenaroma gemischt und zu 2,5 g schweren Brausetabletten verpreßt.

| | |
|---|---|
| Bruchfestigkeit: | 60-80 N. |

### Ergebnis:

In 150 ml Wasser von 16-18°C zerfallen die Tabletten innerhalb 2-2,5 Minuten unter starker Ausfällung von Ibuprofen. An der Wasseroberfläche bildet sich eine zusammenhängende verklebte Schicht von ausgefallener kristalliner oder halbkristalliner Ibuprofensäure. Trinkt man nach dem Zerfall der Tablette diese Kristallsuspension, entsteht das bekannte, sehr unangenehme Brennen an den Mundschleimhäuten, veursacht durch die ungelöste Ibuprofensäure. Rührt man die Kristallsuspension ca. 7 Minuten, so entsteht eine klare Lösung mit einem pH-Wert von 7,1-7,3.

### Bewertung:

Die Brausetablette zerfällt wie angegeben in ca. 2 Minuten. Es entsteht eine klare Lösung, jedoch mit einer erheblichen, untolerablen Menge an ausgefällter Ibuprofensäure, welche auf der Wasseroberfläche schwimmt. Diese Kristallsuspension ist nicht trinkbar.

Eine Auflösung der ausgefällten Ibuprofensäure unter Rühren nach ca. 7 Minuten ist nicht akzeptierbar.

Obwohl sich das Granulat vor der Zugabe der Zitronensäure vollkommen klar auflöst, führt die Zugabe von Zitronensäure zu einer spontanen, offensichtlich unvermeidbaren Ausfällung von bereits gelöstem Ibuprofen.

Insgesamt ergibt sich aus den Vergleichsbeispielen, daß nur mit Hilfe der erfindungsgemäßen Brausezubereitung eine klare, trinkfähige Ibuprofenlösung erzielt werden kann. Mit Hilfe der erfindungsgemäßen Maßnahmen war es somit erstmalig möglich, die saure Wirkung der Zitronensäure des Brausekörpers unter anderem durch folgende Maßnahmen zu beheben:
- Die Zitronensäure muß direkt mit der Base Natriumhydrogencarbonat gemischt und daraus ein separates Brausekörper-Granulat hergestellt werden.
- Das Natriumbicarbonat muß die Hauptmenge der Zitronensäure bei der Auflösung des Brausekörper-Granulates abpuffern, bevor große Mengen an Ibuprofen aus dem Wirkstoffgranulat in Lösung gehen, d.h. erfindungsgemäß wird ein zeitlich versetzt sich auflösendes Ibuprofen-Granulat und ein rascher sich auflösendes Brausekörper-Granulat erzielt.

## Patentansprüche

1. Ibuprofen-Brausezubereitung enthaltend zwei getrennt hergestellte Granulate:
(a) ein Ibuprofengranulat, hergestellt aus Ibuprofensäure und einem basischen Hilfsstoff in einem Mol-Verhältnis von einem Mol Ibuprofensäure auf 0,5 bis 10 Mol basischen Hilfsstoffs, wobei mindestens 70% des basischen Ibuprofengranulats (a) in einer Korngröße im Bereich zwischen 0,1 und 2,0 mm vorliegen, und
(b) ein Ibuprofen-freies Brausegranulat, enthaltend eine Säurekomponente und eine Kohlendioxid bildende Komponente,
wobei die Brausezubereitung ein Mol-Verhältnis von Ibuprofensäure zu der Säurekomponente des Brausegranulats (b) im Bereich von einem Mol Ibuprofensäure auf 0,5 bis 6 Mol Säurekomponente aufweist und das Mol-Verhältnis der Ibuprofensäure zu der Kohlendioxid bildenden Komponente des Brausegranulats (b) im Bereich von einem Mol Ibuprofensäure auf 1 bis 30 Mol der Kohlendioxid-bildenden Komponente liegt, und
wobei sich das Brausegranulat (b) bei Zerfall der Ibuprofen-Brausezubereitung sofort in Wasser auflöst, um Ibuprofengranulatteilchen (a) freizugeben, aus welchen nachfolgend das Ibuprofen in Lösung geht.

2. Brausezubereitung nach Anspruch 1, dadurch gekennzeichnet, daß das Ibuprofen als Racemat, als racemisches Gemisch mit seinen Enantiomeren, als Pseudoracemat, d.h. als Mischung von gleichen Anteilen S- und R-Ibuprofen oder in Mischung unterschiedlicher Anteile von S- und R-lbuprofen im Bereich zwischen S- und reinem R-lbuprofen vorliegt.

3. Brausezubereitung nach Anspruch 1, dadurch gekennzeichnet, daß das Ibuprofengranulat (a) mindestens einen basischen Hilfsstoff enthält, ausgewählt aus der Gruppe, die aus Natriumcarbonat, Kaliumcarbonat, Natriumhydrogencarbonat, Kaliumhydrogencarbonat, Calciumoxid, Calciumhydroxid, Magnesiumoxid, Magnesiumhydroxid, Magnesiumcarbonat, Trinatriumphosphat, Trikaliumphosphat, und Gemischen der sogenannten Verbindungen besteht.

4. Brausezubereitung nach Anspruch 3, dadurch gekennzeichnet, daß der Hilfsstoff Natriumcarbonat und/oder Kaliumcarbonat ist.

5. Brausezubereitung nach Anspruch 1, dadurch gekennzeichnet, daß die Säurekomponente ausgewählt ist aus der Gruppe die aus Weinsäure, Zitronensäure, Ascorbinsäure, Apfelsäure, Adipinsäure, Natriumdihydrogencitrat und deren Gemischen besteht.

6. Brausezubereitung gemäß Anspruch 5, dadurch gekennzeichnet, daß die Säurekomponente Natriumdihydrogencitrat ist.

7. Brausezubereitung nach Anspruch 1, dadurch gekennzeichnet, daß die Kohlendioxid bildende Komponente ausgewählt ist aus der Gruppe, die aus Natriumbicarbonat, Natriumcarbonat, Kaliumbicarbonat, Kaliumcarbonat, Natriumglycincarbonat und Mischungen der vorgenannten Verbindungen, besteht.

8. Brausezubereitung gemäß Anspruch 7, dadurch gekennzeichnet, daß die Kohlendioxid bildende Komponente Natriumbicarbonatund/oder Kaliumbicarbonat ist.

9. Brausezubereitung nach Anspruch 2, die in Form einer Brausetablette vorliegt, dadurch gekennzeichnet, daß sie 200 bis 800 mg racemisches Ibuprofen oder 100 bis 400 mg S-Ibuprofen enthält.

10. Brausezubereitung gemäß Anspruch 9, dadurch gekennzeichnet, daß die Brausetablette 200 bis 400 mg racemisches Ibuprofen enthält.

11. Brausezubereitung gemäß Anspruch 2, die in Form von in Sachets abgefülltem Granulat vorliegt, dadurch gekennzeichnet, daß sie 200 bis 800 mg racemisches Ibuprofen oder 100 bis 400 mg S-Ibuprofen enthält.

12. Brausezubereitung nach Anspruch 11, dadurch gekennzeichnet, daß die Sachets 200 bis 800 mg racemisches Ibuprofen enthält.

13. Brausezubereitung nach Anspruch 1, dadurch gekennzeichnet, daß mindestens 70% des basischen Ibuprofengranulats (a) in einer Korngröße im Bereich zwischen 0,1 und 1,25 mm vorliegen.

14. Brausezubereitung nach Anspruch 1, dadurch gekennzeichnet, daß das Ibuprofengranulat (a) eine Auflösegeschwindigkeit von 50 bis 200 Sek. aufweist.

15. Brausezubereitung nach Anspruch 1, dadurch gekennzeichnet, daß nach Auflösen von 100 bis 400 mg S-Ibuprofen oder 200 bis 800 mg racemischem Ibuprofen der entsprechenden Menge der Brausezubereitung in 150 ml Wasser der pH-Wert zwischen 6,5 und 9,0 beträgt.

16. Brausezubereitung nach Anspruch 1, dadurch gekennzeichnet, daß das Wirkstoffgranulat (a) an sich bekannte Hilfsstoffe zur Steuerung der Auflösegeschwindigkeit enthält.

17. Brausezubereitung nach Anspruch 1, dadurch gekennzeichnet, daß sie weitere Hilfsstoffe wie Süßstoffe und/oder Zuckeraustauschstoffe und/oder Aromen enthält.

18. Verfahren zur Herstellung einer Ibuprofen-Brausezubereitung gemäß einem der Ansprüche 1 bis 17, dadurch gekennzeichnet, daß man aus Ibuprofen-säure und mindestens einem basischen Hilfsstoff und gegebenenfalls weiteren wasserlöslichen Hilfsstoffen ein Ibuprofengranulat (a) herstellt, separat ein Ibuprofen-freies Brausekörpergranulat (b), enthaltend eine Säurekomponente und eine Kohlendioxid-bildende Komponente, herstellt, die beiden Komponenten (a) und (b) mit üblichen weiteren Hilfsstoffen vermischt, zu Brausetabletten verpreßt oder die Brausezubereitung in Sachets abfüllt.

## Claims

1. Effervescent ibuprofen preparation containing two separately produced granulates:
(a) an ibuprofen granulate, produced from ibuprofen acid and a basic adjuvant in a molar ratio of one mol ibuprofen acid to between 0.5 and 10 mol basic adjuvant, at least 70 % of the basic ibuprofen granulate (a) being present in a granular size in the range between 0.1 and 2.0 mm, and
(b) an effervescent ibuprofen-free granulate, containing an acid component and a component which forms carbon dioxide,
the effervescent preparation having a molar ratio of ibuprofen acid to the acid component of the effervescent granulate (b) in the range of one mol ibuprofen acid to between 0.5 and 6 mol acid component, and the molar ratio of the ibuprofen acid to the component of the effervescent granulate (b) which forms carbon dioxide lying in the range of one mol ibuprofen acid to between 1 and 30 mol of the component which forms carbon dioxide, and
the effervescent granulate (b) dissolving immediately in water with the disintegration of the effervescent ibuprofen preparation, in order to release ibuprofen granulate particles (a), from which the ibuprofen subsequently separates.

2. Effervescent preparation according to claim 1, characterised in that the ibuprofen is present as a racemate, as a racemic mixture with its enantiomers, as a pseudoracemate, i.e. as a mixture of equal proportions of S- and R-ibuprofen or in a mixture of different proportions of S- and R-ibuprofen in the range between S- and pure R-ibuprofen.

3. Effervescent preparation according to claim 1, characterised in that the ibuprofen granulate (a) contains at least one basic adjuvant, selected from the group which includes sodium carbonate, potassium carbonate, sodium hydrogen carbonate, potassium hydrogen carbonate, calcium oxide, calcium hydroxide, magnesium oxide, magnesium hydroxide, magnesium carbonate, trisodium phosphate, tripotassium phosphate and mixtures of the aforementioned compounds.

4. Effervescent preparation according to claim 3, characterised in that the adjuvant is sodium carbonate and/or potassium carbonate.

5. Effervescent preparation according to claim 1, characterised in that the acid component is selected from the group which includes tartaric acid, citric acid, ascorbic acid, malic acid, adipic acid, sodium dihydrogen citrate and mixtures thereof.

6. Effervescent preparation according to claim 5, characterised in that the acid component is sodium dihydrogen citrate.

7. Effervescent preparation according to claim 1, characterised in that the component which forms carbon dioxide is selected from the group which includes sodium bicarbonate, sodium carbonate, potassium bicarbonate, potassium carbonate, sodium glycine carbonate and mixtures of the aforementioned compounds.

8. Effervescent preparation according to claim 7, characterised in that the component which forms carbon dioxide is sodium bicarbonate and/or potassium bicarbonate.

9. Effervescent preparation according to claim 2, which is present in the form of an effervescent tablet, characterised in that it contains between 200 and 800 mg racemic ibuprofen or between 100 and 400 mg S-ibuprofen.

10. Effervescent preparation according to claim 9, characterised in that the effervescent tablet contains between 200 and 400 mg racemic ibuprofen.

11. Effervescent preparation according to claim 2, which is present in the form of granulate decanted into sachets, characterised in that it contains between 200 and 800 mg racemic ibuprofen or between 100 and 400 mg S-ibuprofen.

12. Effervescent preparation according to claim 11, characterised in that the sachets contain between 200 and 800 mg racemic ibuprofen.

13. Effervescent preparation according to claim 1, characterised in that at least 70 % of the basic ibuprofen granulate (a) is present in a granular size in the range between 0.1 and 1.25 mm.

14. Effervescent preparation according to claim 1, characterised in that the ibuprofen granulate (a) has a dissolving rate of between 50 and 200 sec.

15. Effervescent preparation according to claim 1, characterised in that, after 100 to 400 mg S-ibuprofen or 200 to 800 mg racemic ibuprofen of the corresponding quantity of the effervescent preparation have been dissolved in 150 ml water, the pH value is between 6.5 and 9.0.

16. Effervescent preparation according to claim 1, characterised in that the additive granulate (a) contains adjuvants, known per se, for controlling the dissolving rate.

17. Effervescent preparation according to claim 1, characterised in that it contains additional adjuvants, such as sweetening agents and/or sugar-substitutes and/or flavours.

18. Method of producing an effervescent ibuprofen preparation according to one of claims 1 to 17, characterised in that an ibuprofen granulate (a) is produced from ibuprofen acid and at least one basic adjuvant and possibly additional water-soluble adjuvants, an ibuprofen-free effervescent body granulate (b) is produced separately, containing an acid component and a component which forms carbon dioxide, the two components (a) and (b) are mixed with conventional additional adjuvants and compressed to form effervescent tablets, or the effervescent preparation is decanted into sachets.

## Revendications

1. Préparation effervescente à base d'ibuprofen comprenant deux granulats obtenus de façon séparée:
a) un granulat d'ibuprofen, obtenu à partir d'ibuprofen sous forme acide et d'un adjuvant basique dans des proportions molaires telles que l'on aie 1 mole d'ibuprofen sous forme acide pour 0,5 jusqu'à 10 moles d'adjuvant basique, et pour lequel au moins 70% du granulat basique d'ibuprofen (a) a une taille de granules comprise dans un intervalle de 0,1 à 2,0 mm, et
b) un granulat effervescent exempt d'ibuprofen, qui comprend un composé acide et un composé générateur de dioxyde de carbone,
telle que la préparation effervescente indique une proportion molaire d'ibuprofen sous forme acide vis à vis du composé acide du granulat effervescent (b) comprise dans un intervalle de 1 mole d'ibuprofen sous forme acide pour 0,5 jusqu'à 6 moles de composé acide et une proportion molaire d'ibuprofen sous forme acide vis à vis du composé générateur de dioxyde de carbone du granulat effervescent (b) comprise dans un intervalle de 1 mole d'ibuprofen sous forme acide pour 1 jusqu'à 30 moles de composé générateur de dioxyde de carbone, et
telle que lors de la décomposition de la préparation effervescente à base d'ibuprofen le granulat effervescent (b) se dissolve immédiatement dans l'eau pour libérer des petits fragments de granulat d'ibuprofen (a) à partir desquels l'ibuprofen va se dissoudre.

2. Préparation effervescente selon la revendication 1, caractérisée en ce que l'ibuprofen se trouve sous forme de racémate, de mélange racémique avec ses énantiomères, de pseudo-racémate, c'est à dire de mélange en des proportions équivalentes de S- et R-ibuprofen ou de mélange en des proportions différentes de S- et R-ibuprofen compris entre le S- et le pur R-ibuprofen.

3. Préparation effervescente selon la revendication 1, caractérisée en ce que le granulat d'ibuprofen (a) comprend au moins un adjuvant basique sélectionné parmi le groupe constitué par le carbonate de sodium, le carbonate de potassium, l'hydrogénocarbonate de sodium, l'hydrogénocarbonate de potassium, l'oxyde de calcium, l'hydroxyde de calcium, l'oxyde de magnésium, l'hydroxyde de magnésium, le carbonate de magnésium, le sodiumtriphosphate, le potassiumtriphosphate, et les mélanges desdits composés.

4. Préparation effervescente selon la revendication 3, caractérisée en ce que l'adjuvant est du carbonate de sodium et/ou du carbonate de potassium.

5. Préparation effervescente selon la revendication 1, caractérisée en ce que le composé acide est sélectionné parmi le groupe constitué par l'acide acétique, l'acide citrique, l'acide ascorbique, l'acide malique, l'acide adipique, le citrate de sodium dihydrogéné, et les mélanges desdits composés.

6. Préparation effervescente selon la revendication 5, caractérisée en ce que le composé acide est du citrate de sodium dihydrogéné.

7. Préparation effervescente selon la revendication 1, caractérisée en ce que le composé générateur de dioxyde de carbone est sélectionné parmi le groupe constitué par le bicarbonate de sodium, le carbonate de sodium, le bicarbonate de potassium, le carbonate de potassium, le glycincarbonate de sodium, et les mélanges desdits composés.

8. Préparation effervescente selon la revendication 7, caractérisée en ce que le composé générateur de dioxyde de carbone est du bicarbonate de sodium et/ou du bicarbonate de potassium.

9. Préparation effervescente selon la revendication 2, qui se trouve sous la forme d'un comprimé effervescent, caractérisée en ce qu'elle contient 200 à 800 mg d'ibuprofen racémique ou 100 à 400 mg de S-ibuprofen.

10. Préparation effervescente selon la revendication 9, caractérisée en ce qu'elle contient 200 à 400 mg d'ibuprofen racémique.

11. Préparation effervescente selon la revendication 2, - qui se trouve sous la forme de granules présents dans des sachets, caractérisée en ce qu'elle contient 200 à 800 mg d'ibuprofen racémique ou 100 à 400 mg de S-ibuprofen.

12. Préparation effervescente selon la revendication 11, caractérisée en ce qu'elle contient 200 à 800 mg d'ibuprofen racémique.

13. Préparation effervescente selon la revendication 1, caractérisée en ce que au moins 70% du granulat basique d'ibuprofen (a) a une taille de granules comprise dans un intervalle de 0,1 à 1,25 mm.

14. Préparation effervescente selon la revendication 1, caractérisée en ce que le granulat d'ibuprofen (a) présente une vitesse de dissolution de 50 à 200 secondes.

15. Préparation effervescente selon la revendication 1, caractérisée en ce que après dissolution dans 150 ml d'eau de la quantité de préparation effervescente correspondant à 100 à 400 mg de S-ibuprofen ou 200 à 800 mg d'ibuprofen racémique, la valeur de pH se situe entre 6,5 et 9,0.

16. Préparation effervescente selon la revendication 1, caractérisée en ce que le granulat qui contient la substance active (a) comprend des adjuvants connus en soi pour guider la vitesse de dissolution.

17. Préparation effervescente selon la revendication 1, caractérisée en ce qu'elle comprend additionellement des adjuvants tels que des agents sucrants et/ou des agents edulcorants de substitution et/ou des arômes.

18. Procédé de préparation d'une préparation effervescente selon l'une quelconque des revendications 1 à 17, caractérisé en ce que l'on produit un granulat d'ibuprofen (a) à partir d'ibuprofen sous forme acide et d'au moins un adjuvant basique et optionellement d'autres adjuvants solubles dans l'eau, en ce que l'on produit de façon séparée un granulat effervescent exempt d'ibuprofen (b) qui comprend un composé acide et un composé générateur de dioxyde de carbone, en ce que l'on mélange les deux composés (a) et (b) avec d'autres adjuvants communément utilisés, et en ce que l'on presse l'ensemble sous la forme d'un comprimé ou que l'on remplit des sachets avec ladite préparation effervescente.
